# EUROPEAN PATENT SPECIFICATION

(11) **EP 3 986 501 B1**
(45) Date of publication and mention of the grant of the patent: **21.05.2025**
(21) Application number: 20740383.3
(22) Date of filing: 17.06.2020
(51) Int. Cl.: A61M 1/36

(54) **FILTER ASSEMBLY AND CONTAINER FOR COLLECTING A BODY FLUID CONTAINING THE SAME**
FILTERANORDNUNG UND BEHÄLTER ZUM SAMMELN EINER KÖRPERFLÜSSIGKEIT DAMIT
ENSEMBLE FILTRE ET RÉCIPIENT POUR COLLECTER UN FLUIDE CORPOREL LE CONTENANT

(30) Priority: 19.06.2019 EP 19181285
(43) Date of publication of application: 27.04.2022
(73) Proprietor: Fresenius Hemocare Italia S.r.l., 41037 Mirandola (MO) (IT)
(72) Inventor: ZAMBIANCHI, Laura, 42124 Reggio Emilio (IT); BOSELLI, Matteo, 41038 San Felice Sul Panaro (IT)
(74) Representative: Maikowski & Ninnemann Patentanwälte Partnerschaft mbB
(86) International application number: PCT/IB2020/000469
(87) International publication number: WO 2020/254871

(56) References cited:
- US-A1- 2011 247 502
- US-B2- 6 908 446

## Description

The present invention relates to a filter assembly, to an appropriate use of such a filter assembly, and to a container for collecting blood comprising such filter assembly.

### Description

Filtering assemblies known from prior art are used to filter different types of fluids, e.g. body fluids, for removing aggregates, particles or specific cells from said fluids. A particular appropriate application of filter assemblies is the filtration of blood. Whole blood or blood components may be separated and further processed for a variety of uses, particularly for use as transfusion products.

Filtering assemblies are also used in auto transfusion processes during surgeries procedures; i.e. blood of a patient is recovered during surgery and re-infused into the patient. This is also known as intraoperative blood salvage (IOBS), or autologous blood transfusion or cell salvage. It has been used for many years and gained greater attention over time as risks associated with allogenic (separate-donor) blood transfusion have seen greater publicity and become more fully appreciated. Several medical devices have been developed to assist in salvaging the patient's own blood in the perioperative setting. The procedure is frequently used in cardiothoracic and vascular surgery, during which blood usage has traditionally been high.

Whole blood recovered during surgery, but being exposed to surgery, air etc. has different characteristics than blood collected in a blood bank from a donor for allogeneic transfusion purpose. For example, blood salvage requires a removal of blood clots, non-cellular substances, such as drugs or fluids used during surgery, bone fragments and surgery debris.

Autologous blood recycling or intraoperative blood salvage is often accompanied by foam formation. Blood foam formation (sometimes referred to as "skimming") is related to collection or transportation of blood in presence of air in large tubes (e.g. collected with Yankauer cannulae) and thus related to turbulence in the suction process.

One approach to avoid foam formation in shed blood is the use of antifoam agents in reservoirs where shed blood is collected for autotransfusion or extracorporeal circulation. In the past polydimethylsiloxane (PDMS) - hydrophobed silica was widely used as antifoam. However, PDMS may be partially leached into the blood, where it emulsifies. In IOBS, it is presumed that most of it will be found in the waste after the washing procedure, but it cannot be excluded that part of it is found in the Red Cell Concentrate that is autotransfused to the patient. Even though PDMS-hydrophobed silica is non-toxic it was associated as a possible source for emboli due to capillaries blockage and postoperative deaths and is no longer used as antifoam. Thus, nowadays only PDMS without silica is used. The defoaming principle remains the same for PDMS, while the physical way of action, a silica particle that "breaks" the bubble is no longer applied for medical devices, but is still used in non-medical defoaming.
Another approach is the use of defoaming layers. US 6,908,446 B2 discloses a blood reservoir that is able to reducing damage to blood storage. In particular, the container for collection of blood comprises a filter assembly which in turn comprises a defoaming layer. US 2011/0247502 A1 discloses a container for collection of blood comprising two filter assernblies and a filter holder which contacts and supports the filter assemblies, wherein the first filter assembly comprises a defoaming layer, and the second one comprises a non-woven prefilter layer and a mesh layer.

However, there is still a need for alternative antifoaming concepts.

It is an object of the present invention to provide a filter system that allow a reduction or even prevention of foam formation in filtered body fluids, such as shed blood, and at the same time reduce the risk of leaching chemicals into the filtered body fluid.

This object is achieved by a filter assembly having the features explained in the following.

Accordingly, a filter assembly for filtering blood as body fluid comprising a filter system and a filter holder is provided, wherein the filter system and the filter holder are in contact with each other or are connected to each other.

The filter system consists of at least two layers,
- wherein the first layer is a defoaming layer made of a monofilament woven open mesh fabric and with an embossed three dimensional structure configured for entrapping foam built up in the blood ,
- wherein the second layer is a mesh filter layer, and
- wherein the mesh filter layer is arranged downstream of the defoaming layer.

The terms "downstream" and "upstream" refer to a flow direction of a fluid to be filtered by the filter assembly. Thus, the fluid to be filtered contacts at first the defoaming layer followed by the mesh filter layer.

Such a filter assembly can well be used for filtering blood and other body fluids. If such a filter assembly is used, foam formation in the blood and in other body fluids is effectively prevented. The present filter assembly allows to remove foam mechanically. Thereby, no anti-foaming agents are necessary so that the risk of leaching chemicals into the filtrated body fluid is fully avoided. The present filter assembly is in particular useful for an autotransfusion reservoir or container.

It was surprisingly found that the use of a defoaming layer with an embossed 3D structure removes or reduces foam. The underlying mechanism may be an entrapment of the foam within the defoaming layer. The spatial 3Dstructure allows for an entrapment of gas bubbles formed in the body fluid; i.e. gas bubbles are entrapped within the spatial structure. Another mechanism could be that the bubbles of the foam are blown up when the pass through the downstream mesh filter.

The embossed 3D structure may be in form of a diamond pattern with regularly arranged protrusions and depressions of a certain height. One can describe the spatial pattern also as a zig-zag-structure or pyramid like structure.

As mentioned previously, the defoaming layer is a structured open mesh fabric.

In an embodiment the defoaming layer comprises a mesh opening between 100 - 500 µm, in particular between 150 - 400 µm, in particular between 200 - 350 µm, and more particular between 250-300 µm, for example 250 µm.

The mesh count (typically the number of threads per cm) of the open mesh fabric forming the defoaming layer may be between 10-50 n/cm, in particular between 10-30 n/cm, more particular between 10-20 n/cm, such as 12-16 n/cm.

The fabric of the defoaming layer is made of monofilament fibers with a diameter between 100-350 µm, in particular 150 - 300 µm, more particular 200-300 µm, such as 200, 250, 300 µm. The monofilament fibers can be made of any thermoplastic material, but preferably of polypropylene (PP), polyethylene (PE), polyether ether ketone (PEEK) and others. Polypropylene is the most preferred one.

The defoaming layer can have a weight of 50-250 g/m2, in particular 100-200 g/cm2, more particular 150-200 g/cm2, such as 98-103 g/cm2, 180-190 g/cm2, 156 g/cm2.

The thickness of the defoaming layer may be 150-650 µm, in particular 200 - 500 µm, in particular 250- 400 µm, in particular 300 - 350 µm.

The defoaming layer may also be of a sponge-like structure, for example made of polyurethane or polyester foam.

In an embodiment, the first mesh layer is made of a plurality of interconnecting threads forming a grid or a net. Thus, vertically arranged threads and horizontally arranged threads are connected to each other at connecting points so as to form a grid.

In an embodiment, the threads or filaments of the mesh filter layer have a circular cross-section. However, other cross sections, such as an elliptic, a rectangular, a quadratic or a triangular cross-section would also be possible. Likewise, mixtures of threads or filaments having different cross sections are also possible.

In an embodiment, the mesh size of the mesh filter layer is in a range between 20 and 160 µm, in particular between 30 and 140 µm, in particular between 40 and 130 µm, in particular between 50 and 120 µm, in particular between 60 and 110 µm, in particular between 70 and 100 µm, in particular between 80 and 90 µm. Ranges of 100 to 130 µm, 105 to 125 µm, 70 to 90 µm, 75 to 85 µm, 30 to 45 µm and 35 to 40 µm are particularly appropriate.

In an embodiment, the mesh filter layer can comprise or can be entirely made of a polymer such as a polyester, polyethylene, polypropylene, polybutylene, polymethylpentene, polyethylene terephthalate, polytrimethylene terephthalate, polybutylene terephthalate, poly(butylene terephthalate-co-polyalkylene glycol terephthalate), nylon 6,6, nylon 6,9, nylon 6/12, nylon 11, nylon 12, cellulose acetate, cellulose acetate propionate, or a combination thereof. Thereby, a non-hydrophobic or hydrophilic material is particularly appropriate to produce the mesh filter layer. It is also possible to increase the hydrophilicity of the material which is used to produce the mesh filter layer or to increase the hydrophilicity of the already produced filter. Thereby, a physical treatment is more appropriate than the deposition of chemicals because such chemicals might potentially be leached from the mesh filter during use of the container.

An appropriate surface area of the mesh filter layer is in a range of 300 to 1000 cm², in particular 400 to 900 cm², in particular 500 to 800 cm², in particular 600 to 700 cm².

In a further, preferred embodiment the filter assembly comprises a prefilter layer (depth filter) arranged downstream of the defoaming layer and upstream of the mesh filter layer; i.e. sandwiched between defoaming layer and mesh filter layer.

The prefilter layer comprises or essentially consists of a non-woven fabric of fibers. Thereby, the fibers are arranged such that gaps are formed between the randomly deposited fibers. The gaps can be defined as openings with an average pore size. Consequently, a pore size of the non-woven fabric results.

The pore size of the prefilter material is smaller than the upstream defoaming layer but larger than the downstream mesh filter material.

In an embodiment, the prefilter layer comprises or essentially consists of a spunbond nonwoven fabric. The individual fibers of this fabric may have any desired cross-section, such as a circular, elliptic rectangular, quadratic or triangular cross-section. Mixtures of fibers having different cross sections are also possible.

The fibers of the prefilter layer can also generally have any shape. However, it turned out that particularly good filtering can be achieved if the fibers, or at least a part of the fibers, comprise at least one groove extending in the longitudinal direction of the respective fiber. To give an example, the fibers may comprise three grooves each extending in a longitudinal direction of the fiber. Then, aggregates, fat and/or platelets can be particularly well filter from blood or another body fluid flowing through the filter assembly.

In an embodiment, at least a part of the fibers has a lobate cross-section. Such a lobate cross-section can be achieved, in an embodiment, by forming a groove in the fibers in the longitudinal direction. A trilobal cross-section is a particularly well-suited example of a lobate structure. Such trilobal fibers are generally known, e.g., from WO 2013/110694 A1, the entire content of which is hereby incorporated by reference.

The fibers making up the non-woven fabric of the prefilter layer can be, in an embodiment, spunbond fibers or melt-blown fibers. While spunbond fibers typically have a fiber diameter that is at least 20 µm or larger, melt-blown fibers may have lower diameters of less than 20 µm.

The prefilter layer comprises or essentially consists of continuous filament spunbond nonwoven fabric. Said fabrics are obtained in continuous filament nonwoven processes (meltblowing and spunbonding) that start extruding chips or pellets of raw material. The length of the filament is theoretically infinite.

The fibers of the prefilter material may be monocomponent, bicomponent or multicomponent fibers, including "island in the sea" fibers. The fibers may consist of one polymer or a blend of polymers. Suitable materials for the fibers are, for example, a polyester, polyethylene, polypropylene, polybutylene, polymethylpentene, polyethylene terephthalate, polytrimethylene terephthalate, polybutylene terephthalate, poly(butylene terephthalate-co-polyalkylene glycol terephthalate), nylon 6,6, nylon 6,9, nylon 6/12, nylon 11, nylon 12, cellulose acetate, cellulose acetate propionate, or a combination thereof. Thereby, a non-hydrophobic or hydrophilic material is particularly appropriate to produce the fibers. It is also possible to increase the hydrophilicity of the material which is used to produce the fibers or to increase the hydrophilicity of the already produced fibers. Thereby, a physical treatment is more appropriate than the deposition of chemicals because such chemicals might potentially be leached from the fibers or the fabric produced therefrom during use of the filter assembly.

In an embodiment, the pore size of the prefilter layer is in a range of between 20 and 150 µm, in particular between 30 and 140 µm, in particular between 40 and 130 µm, in particular between 50 and 120 µm, in particular between 60 and 110 µm, in particular between 70 and 100 µm, in particular between 80 and 90µm. Ranges of 100 to 130 µm, 105 to 125 µm, 70 to 90 µm, 75 to 85 µm, 30 to 45 µm and 35 to 40 µm are particularly appropriate.

As mentioned above the filter system may comprise two layers orthree layers.

In case of the two-layer system the first layer is a defoaming layer and the second layer is a mesh filter layer. Such a two layer system may be used for large pore size filtration. The mesh size of the mesh filter may be 120 µm, with 50% of open permeability and 34% of closed permeability,

In case of a three-layer system the first layer is a defoaming layer, the second (sandwiched) layer is prefilter layer (depth filter material) and the third layer is a mesh filter layer. Such a three-layer system may be used for low pore size filtration. The mesh size of the mesh filter layer may be 40µm, with 40% of open permeability and 12% of very closed permeability,

In a specifically preferred embodiment the filter system comprises a defoaming layer with a mesh size between 200 and 300 µm, preferably 250 µm, a prefilter layer with a pore size between 50 and 100 µm, preferably 70 µm and a mesh filter layer with a mesh size between 30 and 50 µm, preferably 40 µm.

The filter assembly further comprises a filter holder. This filter holder contacts the filter system of defoaming layer, optional prefilter layer and mesh filter layer. In doing so, it also stabilizes the filter system. Thus, the filter holder serves for keeping the different layers of the filter system in place; it acts as structural support. The filter assembly is a stand-alone filter assembly (self-sustained filter assembly) or it can be one part (overmolded) with filter layers.

The filter holder is made from plastic and is overmolded over at least a part of the filter system. It is preferred, if the overmolding is over all filter material layers of the filter system and not only the outer one. This can be achieved by dipping the filter material in molten plastic. When the plastic cools down, and becomes rigid, a holder structure (a sort of cage) is created that englobes the filter material layers. It tightly connects the layer of the filter system together and serves particularly easy for a good stabilization of the layer of the filter system.

It was surprisingly found that such a filter assembly can well be used for filtering blood and other body fluids. If such a filter assembly is used, foam formation in the blood and in other body fluids is effectively prevented. Thereby, no anti-foaming agents are necessary so that the risk of leaching chemicals into the filtrated body fluid is fully avoided.

In an embodiment, the filter assembly does not comprise any anti-foaming agents. By avoiding the use of such anti-foaming agents the risk of leaching anti-foam chemicals into a fluid that is flowing through the filter assembly is fully prevented. This enhances the quality of the fluid filtered by the filter assembly.

The present invention relates in an aspect to the use of the filter assembly having the features explained above for filtering blood *ex vivo.*

In yet a further aspect the present invention relates to a container for collecting blood comprising a filter assembly as described in detail above as a filter module.

Such a container comprises a container housing with a body fluid inlet that allows a body fluid to enter an inlet section of the container housing. The container housing furthermore comprises a body fluid collection section and a vacuum connector for connecting a vacuum source to the container housing. In doing so, a negative pressure can be applied to the inlet section and to the body fluid collection section. Thereby, the vacuum source is typically connected via a vacuum line to the vacuum connector of the container housing.

Furthermore, the container housing comprises the filter module that separates the inlet section from the body fluid collection section. To be more specific, the filter module is arranged between the inlet section and the body fluid collection section so that a body fluid needs to pass the filter in order to flow from the inlet section to the body fluid collection section. Expressed in other words, the filter module has a raw side and a clean side. The raw side faces the inlet section andthe clean side faces the body fluid collection section.

In particular, the filter assembly as filter module is arranged between the inlet section and the body fluid collection section, such that the defoaming layer faces the inlet section and the mesh filter layer faces the body fluid collection section.

In an embodiment, the container housing comprises ahydrophobic filter (i.e. separate from the filter assembly) that is arranged between the body fluid collection section and the vacuum connector. Thereby, the term "between" relates to a flow direction of air drawn by a vacuum source from the container (or the body fluid collection section of the container housing) during the intended operation of the container. I.e., any fluid that is drawn from the interior of the container housing (in particular air and smoke) needs to pass the hydrophobic filter prior to entering a vacuum line connected to the vacuum connector. Thus, the hydrophobic filter serves as protective element for a vacuum line being connected to the vacuum connector of the container housing, when vacuum is generated by a vacuum pump.

The novel filter module and the hydrophobic filter synergistically act together so that this combination results in the effects explained in the following. By constructing the filter module with a filter holder and a filter assembly comprising a defoaming layer, an optional prefilter layer and a mesh filter layer, much more flexibility is given for the design of the filter holder than in case of using prefabricated filter sockets like in prior art. Furthermore, it is possible to manufacture the filter holder together with the filter assembly in a single manufacturing step, and the connection of the co-molded filter assembly component to the container is easily automatable. This significantly reduces manufacturing costs and increases the performance of the container due to more reliable and reproducible manufacturing steps.

The additional hydrophobic filter arranged in flow direction before the vacuum connector serves both as overfill protection and as smoke filter. Thus, it combines the properties of these elements, which are used in form of individual components according to prior art, in a single element. This single element is thereby included in the container housing. Thus, it is not necessary to connect it with a separate vacuum line. There is no need to clean and/or sterilize the hydrophobic filter. Rather, it can be designed as disposable that is discarded together with the whole container. This additionally facilitates the use of the container.

Using a filter assembly comprising a defoaming layer, an optional prefilter layer and a mesh filter layer instead of a regular filter (made only of fibers or foam or a membrane) is connected to the effect that extremely reproducible filtering conditions can be met. While regular filters have an average pore size with many pores being bigger or smaller than the average pore size, a mesh filter has a clearly defined mesh size that essentially does not vary. Besides, the use of the defoaming layer reduces or even prevents foam formation.

In an embodiment, a top cover or an inlet section of the container housing and the filter holder are manufactured as one piece, i.e., they are integrally formed. This facilitates the manufacturing steps significantly since no manual attachment of a filter to the container housing is necessary anymore. To give an example, the filter holder and (at least parts of) the container housing can be co-molded in one single injection molding step. Then, an outlet of the inlet section of the container housing turns integrally into an inlet of an interior of the filter holder. Any body fluid that enters the inlet section will then flow or will be drawn from the inlet section of the container housing towards an interior space of the filter holder. Ithas then to pass the filter assembly in order to reach the body fluid collection section.

As mentioned previously, the filter holder and the filter assembly are free of antifoam agents. While certain antifoam agents are necessary in case of regular filters to avoid an undesired foaming of blood, the design of the blood path through the filter element tend to induce foam formation in blood only to a very low extent. If no antifoam agents are used, no such agents can be leached into body fluids so that no corresponding contamination of the body fluid needs to be feared.

In an embodiment, the filter holder comprises bars or struts that stabilize the filter material which is located in an interior of the filter holder. Such bars can be easily produced by injection molding, e.g., directly on the filter material. They prevent the filter material to collapse and to stick wet. Furthermore, they can be used during injection molding to carry molten plastics to create a punt at the bottom of the filter element.

In an embodiment, an inlet area of the filter module is funnel-shaped. Such a funnel shape reduces the risk of foam formation in the body fluid. Thus, the funnel shape of the inlet area can also be considered as part of the concept "defoaming by design" that is, in an embodiment, applied to the filter module. Thereby, typically the filter holder has this specific funnel-shaped inlet area, whereas the filter assembly does not need to have any specific design (as long as it fits into the filter holder).

In an embodiment, the bottom of the filter module is not entirely flat, but rather comprises an indention towards an interior space of the filter module. Thereby, this indention extends, in an embodiment, over essentially the full area of the bottom of the filter module (in particular of the filter holder). Then, the bottom of the filter module has a concave shape when looked from the outside of the filter module and a convex shape when looked from the inside of the filter module. Such a design of the bottom of the filter module plays also a role in the "defoaming by design" approach taken in an embodiment. The indention of the bottom of the filter module can also be described as a shape like a champagne's bottle punt. This shape avoids spurts of body fluid passing through the filter module and reduces the falling height of the body fluid passing through the filter module. The lower the falling height, the lower the risk of foam formation in the body fluid.

In an embodiment, the hydrophobic filter is integrated into the top cover of the container housing. Then, it is arranged in an upper location of the container so that the risk of getting into contact with the body fluid is significantly reduced. Furthermore, such integration in the top cover of the container still allows a compact design of the whole container.

The hydrophobic filter comprises, in an embodiment, a filter housing and a filter material placed in the filter housing. While it would be generally possible to design the hydrophobic filter as exchangeable element, it is intended, in an embodiment, that the hydrophobic filter is a disposable element that is discarded together with the whole body fluid collecting container. The lifetime of the hydrophobic filter is generally longer than the lifetime of the body fluid collecting container so that it is generally not necessary to replace the hydrophobic filter during the intended operation of the body fluid collecting container.

The hydrophobic filter may also be a mesh filter, wherein a mesh size of 2 to 20 µm, in particular 3 to 19 µm, in particular 4 to 18 µm, in particular 5 to 17 µm, in particular 6 to 16 µm, in particular 7 to 15 µm, in particular 8 to 14 µm, in particular 9 to 13 µm, in particular 10 to 12 µm is appropriate.

The hydrophobic filter is intended to filter air drawn by a vacuum source from the container for collecting a body fluid, or, to be more precisely, from the body fluid collecting section of this container. The hydrophobic filter protects the vacuum source from smoke (in particular surgical smoke), particles (such as bone or tissue fragments), and blood and also reduces the load of contaminations of the hydrophobic antibacterial filter that is associated to the pump and thus located downstream from the hydrophobic filter.

In an embodiment, the hydrophobic filter comprises a filter material comprising or consisting of a hydrophobic polymer such as polytetrafluoroethylene (PTFE), in particular expanded PTFE (ePTFE).

In an embodiment, the hydrophobic filter comprises a filter material comprising or consisting of a non-hydrophobic polymer such as polyester (such as PET) treated with a hydrophobic polymer, in particular a hydrophobic PET mesh. Since the contact with blood of this element is occasional and very limited in time (spurts) and due to the position (top cover), the risk that the hydrophobic treatment is leached into the blood is minimal. However, the hydrophobic treatment has to be biocompatible.

In an embodiment, the hydrophobic filter comprises a pleated filter material. By pleating the filter material, the effective filter surface can be increased while not increasing the overall space needed for the hydrophobic filter.

In an embodiment, the pleated filter material has a filter surface area being at least 3 times, in particular at least 4 times, in particular at least 5 times, in particular at least 6 times in particular at least 7 times, in particular at least 8 times as high as the surface area of the filter element that houses the hydrophobic filter. The filter surface area might be 3 to 8 times, in particular 4 to 7 times, in particular 5 to 6 as high as the surface area of the filter element that houses the hydrophobic filter. E.g., if the filter element has a surface area of 5 to 20 cm², the total filter surface might be in a range of 15 to 160 cm². Thus, by such an arrangement it is possible to incorporate a quite big filter surface area in a filter element that has only very low space requirements. This facilitates incorporating the hydrophobic filter into the top cover of the container housing while not increasing the dimensions of the top cover as compared to the top covers known from prior art.

In an embodiment, the container is specifically adapted to receive blood as body fluid. I.e., the body fluid referred to in the present description is, in this embodiment, blood.

The less foam formation in the collected body fluid, the better quality or higher yield of the collective body fluid is achieved. In case of blood as body fluid, lower foam formation results in lower hemolysis and lower platelets activation which in turn result in a higher blood recovery and better quality from the individual shed-blood processing steps. Mechanical stress of red blood cells is one ground of hemolysis. Foam formation may be an indicator of biological stress.

In an aspect, the invention relates to a body fluid collecting arrangement comprising a vacuum source and a container according to the preceding explanations. Thereby, the vacuum source is directly connected to a vacuum connector of the container via a vacuum line. No component parts other than the vacuum line is present between the vacuum source and the container.

Body fluid collecting arrangements known from prior art have the following general setup: body fluid collecting container - vacuum line - overfill protection - smoke connector - smoke filter-vacuum line - vacuum pump. Thus, six connection points need to be established in total. If the vacuum source is directly connected to the connector like in the currently discussed aspect of the present invention, only two connection points need to be established: body fluid collecting container - vacuum line - vacuum source. Thus, the integration of the hydrophobic filter into the container housing of the body fluid collecting container renders a separate overflow protection, a separate smoke connector and a separate smoke filter superfluous. Three separate parts each having two connecting points can be fully skipped when relying on this aspect of the present invention. This significantly reduces the workload for medical staff preparing a body fluid collecting arrangement ready to be used.

In case of massive overflow of the body fluid collecting section of the container housing, it might happen that the collected body fluid passes through the hydrophobic filter. Then, the body fluid might enter the vacuum line that is connected to the vacuum connector of the container housing. In an embodiment, the vacuum line comprises a hydrophobic antibacterial filter to protect the pump. Then, such overflow of small amounts of body fluids will not have any consequences. Rather, some hydrophobic vacuum lines are able to keep working also in the presence of accumulation of contaminations or liquids. A guiding principle is to reduce complexity of assembly of the vacuum line to enhance usability.

In an embodiment, the vacuum line comprises an integrally formed antibacterial filter. Thus, this antibacterial filter does not represent an additional component part, but rather is an integral part of the vacuum line.

In an embodiment, the antibacterial filter is a hydrophobic filter. Then, it can also be used to effectively prevent any liquid that has entered the vacuum line, e.g., due to overfill events of the body fluid collection section of the container housing, from entering into the pump being arranged downstream of the antibacterial filter. It is possible to equip such an antibacterial filter with an additional chamber to accommodate any contaminations of the filter. Then, such chambercan also serve for receiving excessive body fluid drawn through the vacuum line due to an overfill event.

In an embodiment, the antibacterial filter has a pore size or mesh size that is sufficiently small to also filter viruses out of the fluid (in particular air) drawn through the vacuum line. In such a case, the antibacterial filter has also anti-viral properties. Then, it can be denoted as anti-viral filter.

In an aspect, the present invention relates to a method for manufacturing a container according to the preceding explanations. Thereby, a top cover of a container housing of the container and a filter holder of the container are co-molded. I.e., they are manufactured as one piece or, expressed in other words, they are integrally formed or integrally molded. Such a manufacturing process is significantly easier than the manufacturing processes known from prior art. It combines the previous method steps of producing a container housing and subsequently attaching a filter into the container housing into one single manufacturing step, namely a co-molding step. It can be accomplished, e.g., by injection molding.

While it is also possible to co-mold the filter assembly together with the filter holder, other approaches are taken in an embodiment of the manufacturing method. To be more precisely, in this embodiment, the filter assembly is applied (e.g., by molding) into the filter holder after the filter holder has been produced. Thereby, the filter holder itself may be co-molded with the top cover of the container housing. In doing so, it is not necessary to produce different molds for different mesh sizes to be applied for a filter module of the container housing. Rather, in this embodiment, only a single mold is necessary to produce a large number of container housing top covers with an integrally formed filter holder, wherein afterwards different filter assemblies filters can be applied into the filter holder, thus resulting in container housing top covers providing different filter properties (in particular different mesh sizes of the filter assembly).

In an aspect, the present invention relates to medical method for drawing intraoperative cell salvage (ICS) from a patient in need thereof. ICS is typically used for patients undergoing surgery or invasive procedures and is intended to provide those patients with an autotransfusion of blood or blood components. This method comprises the steps explained in the following. First, a blood suction line is connected to a blood inlet of a container for collecting blood. Furthermore, vacuum line is connected to a vacuum connector of this container and to a vacuum source, such as a vacuum pump. In doing so, a low pressure can be applied to an interior of the container, when the vacuum source is activated. The container is a container according to the preceding explanations. Thus, it comprises a container housing with the blood inlet that allows blood to enter an inlet section of the container housing. The container housing furthermore comprises a blood collection section. Thereby, the vacuum source is typically connected via a vacuum line to the vacuum connector of the container housing.

Furthermore, the container housing comprises a filter module that separates the inlet section from the blood collection section. To be more specific, the filter module is arranged between the inlet section and the blood collection section so that the blood drawn from the patient needs to pass the filter in order to flow from the inlet section to the blood collection section. Expressed in other words, the filter module has a raw side and a clean side. The raw side faces the inlet section and the clean side faces the blood collection section.

If all elements are assembled, the vacuum source is activated. Then, blood is drawn from a patient (e.g., during a surgical intervention) through the blood suction line into the receiving section of the blood-collecting canister. It then passes the filter and reaches the blood-collecting section. Afterwards, it can be drawn from the blood-collecting container in order to be further processed and/or auto-transfused to the patient.

In an aspect, the present invention relates to a method for manufacturing a filter assembly according to the preceding explanations. As already mentioned, such a filter assembly comprises a filter holder, defoaming layer, an optional prefiltering layer and a mesh filter layer.

The filter holder is arranged downstream the mesh filter layer. The mesh filter layer, in turn, is arranged downstream of the defoaming layer and optional prefiltering layer.

The method is characterized in that the that the filter holder (which is made from plastic) is overmolded over a part of the filter system of defoaming layer, opt. prefiltering layer and mesh filter layer so that it contacts and stabilizes filter layers of the filter system. Thus, only a single manufacturing step is necessary in order to place the filter holder around the layers of filter material since it is manufactured in situ. This significantly facilitates the manufacturing of the filtering assembly as compared to filtering devices known from prior art. The filter holder can also form the ground area of the filter assembly. Thus, it may form the base of the filter assembly and at the same time embeds the filter material layers near the base to fix them.

In an embodiment, the different layers of the filter system initially form a flat ribbon. This flat ribbon is then brought into the desired shape of the filter assembly. This desired shape can be, e.g., the shape of a cylinder jacket, wherein the cylinder has a circular ground area, an elliptic ground area, a rectangular ground area or a quadratic ground area. The free ends of the shaped ribbon are then connected to each other. The connection of the free ends of the shaped ribbon can be accomplished, in an embodiment, by a welding process. Alternatively, the connection can be formed as a non-welded joint. The given shape is fixed by partially overmolding the layers of the filter system with the filter holder. This manufacturing method of a filter assembly according to an aspect of the present invention is significantly easier than manufacturing techniques applied according to prior art. To be more precise, in prior art regularly a tubular filter arrangement made of two welded ribbons needs to be cut. The according manufacturing steps are significantly more difficult and increase the risk of particle contamination since welding and cutting two tubular ribbons may generate particulates that can be leached into a fluid that passes the filter.

Further details of aspects of the present invention will be explained in the following with respect to exemplary embodiments and accompanying Figures. In the Figures:
Figure 1 is a perspective view of an embodiment of a blood-collecting canister;
Figure 2 is a side view onto the broad side of the canister of Figure 1;
Figure 3 is a detailed view of an upper part of the canister of Figure 1 seen from the narrow side of the canister;
Figure 4A is a schematic view of a first embodiment of the filter system according to the invention; and
Figure 4B is a schematic view of asecond embodiment of the filter system according to the invention.

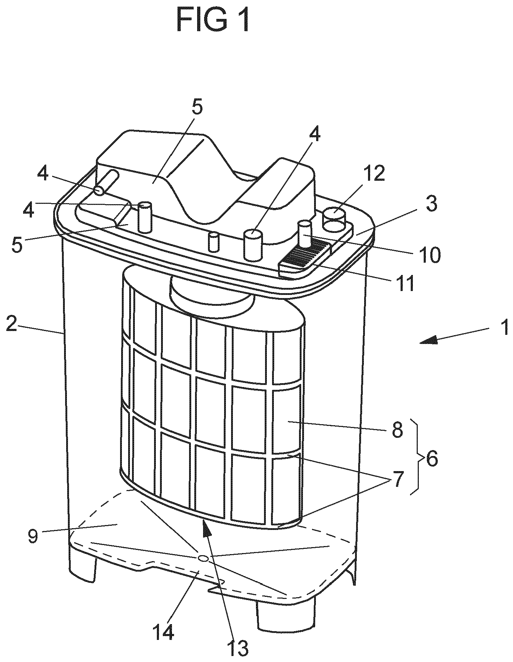

Figure 1 is perspective view of a blood-collecting canister 1 that serves as container for collecting a body fluid. The blood-collecting canister 1 comprises a canister housing 2 having a top cover 3. Three different blood inlets 4 are arranged in the top cover 3. Typically, only one of these blood inlets 4 is used for connecting a blood suction line with the blood-collecting canister 1 in order to draw blood from a patient into the interior of the canister housing 2. The right-most (with reference to Fig. 1) blood inlet 4 is designed as a 3/8 inch connection. The middle blood inlet 4 is designed as luer inlet, wherein the most left blood inlet 4 is designed as suction line connector sized for accommodating typical blood suction lines.

Each of the blood inlets 4 is fluidly connected with a blood receiving section 5 that is arranged on an inner side of the top cover 3. This blood receiving section 5 is in fluid communication with an interior of a filter module 6 (serving as filter assembly) that comprises a skeletal structure 7 that serves as filter holder.

Inside the skeletal structure 7, the material layers of the filter system 8 are arranged. The filter material or filter system 8 comprises a defoaming layer and a mesh filter layer made of a medical grade mesh. If blood enters through the blood inlet 4 into the receiving section 5 of the blood-collecting canister 1, it flows or it is drawn into the interior of the filter module 6. Afterwards, it passes the filter material 8 and reaches a blood collection section 9 of the canister housing 2.

The blood-collecting canister 1 comprises in the top section 3 of the canister housing 2 a vacuum connector 10 that is intended to be connected to a vacuum line and, via the vacuum line, with a vacuum pump that serves as vacuum source. Air or any other gases being present in the blood-collecting section 9that are drawn through the vacuum connector 10 into a connected vacuum line need to pass a hydrophobic filter 11 that is arranged between the blood-collecting section 9 and the vacuum connector 10.

The blood-collecting canister 1 further comprises a safety valve 12 that limits the amount of negative pressure that can be achieved within the interior of the canister housing 2. Thus, the safety valve 12 serves for reducing the risk of the blood-collecting canister 1 to implode due to an undesired low negative pressure in the interior of the canister housing 2.

When seen from the outside, a bottom 13 of the filter module 6 has a concave shape, i.e., it comprises an indention towards the interior of the filter module 6.

Blood that has entered the canister housing 2 through the blood inlet 4 and has passed the filter material 8 collects in the blood-collecting section 9. It can then be drawn through a blood outlet 14 out of the blood-collecting canister 1 in order to be further processed and/or auto-transfused to the patient.

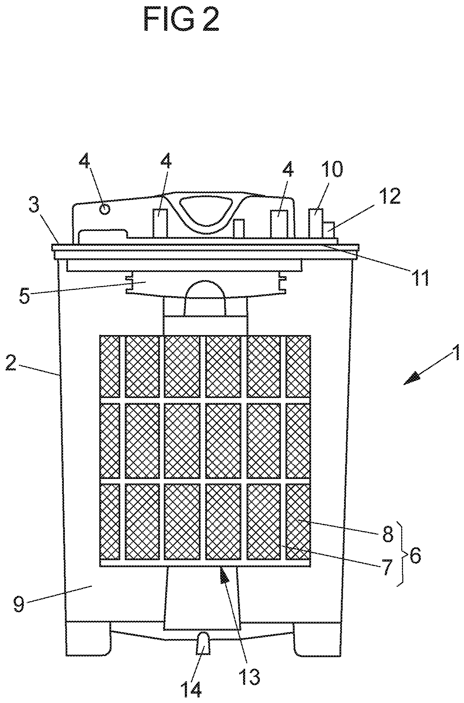

Figure 2 shows the blood-collecting canister 1 from Figure 1 in a side view onto the broad side of the blood-collecting canister 1. Thereby, the same numeral references for the same elements are used. Reference is made to the explanations given with respect to Figure 1. In figure 2, a connection between the filter module 6 and the blood receiving section 5 of the canister housing 2 can be seen. It is apparent from Figure 2 that blood can enter from the blood receiving section 5 only the interior of the filter module 6 and then needs to pass the filter material 8 in order to reach the blood-collecting section 9 of the canister housing 2. Thus, the filter module 6 separates the blood receiving section 5 from the blood-collecting section 9.

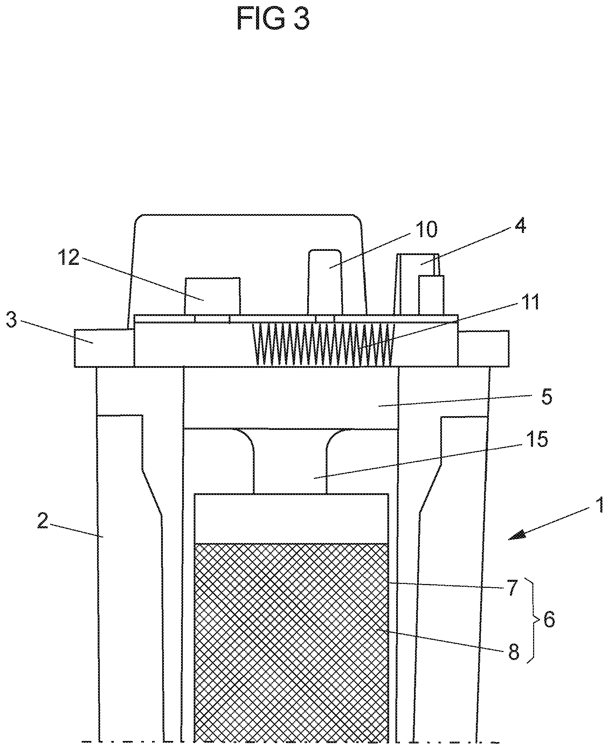

Figure 3 shows a partially cut view from the narrow side onto the blood-collecting canister 1 of Figure 1. Thereby, once again the same numeral references are used for the same elements. Reference is made once again to the explanations given above.

In the depiction of Figure 3, a funnel-shaped inlet 15 is arranged in an inlet area of the filter element can be seen. Blood entering the filter module 6 from the blood receiving section 5 needs to pass needs to pass this funnel-shaped inlet 15. The funnel-shaped inlet 15 serves - together with the filter material 8 and the concavely shaped bottom 13 of the filter module 6 for a reduced foam formation in the blood that passes through the filter module 6. Such a reduced formation of foam leads to blood having better quality than foamed blood and to a higher collection yield due to a lower hemolysis rate.

It can be furthermore seen in the depiction of Figure 3 that the hydrophobic filter 11 comprises a pleated filter material. Due to this folding of the filter material, the effective filter surface area is significantly increased. To give an example, the filter material of the hydrophobic filter 11 has an overall filter surface area of approximately 60 cm². Thereby, the hydrophobic filter itself takes only approximately 10 cm² space in the top cover 3 of the canister housing 2. Thus, by folding the filter material, the effective filter surface area is made six times as big as the surface area needed by the hydrophobic filter element 11.

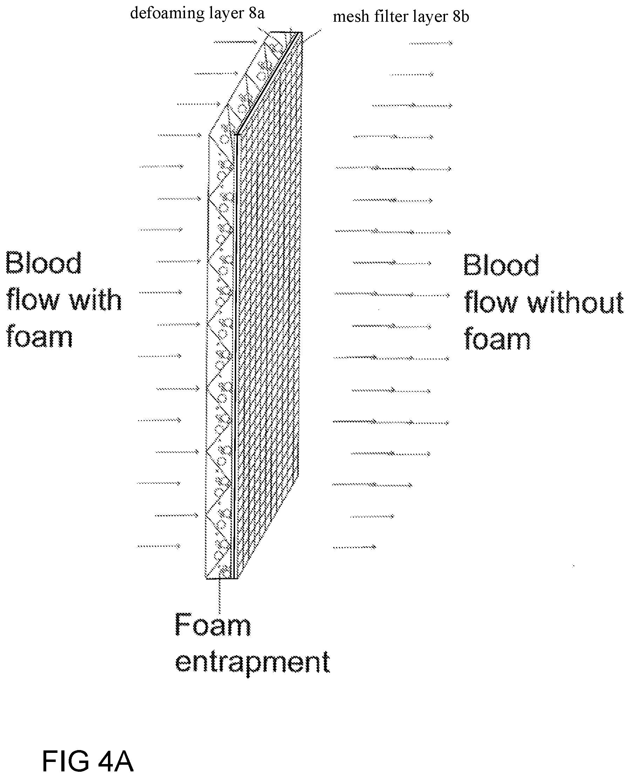

Figure 4A shows a first embodiment of the filter system 8 according to the invention. It shows that the filter system 8 is made a defoaming layer 8a and a mesh layer 8b.

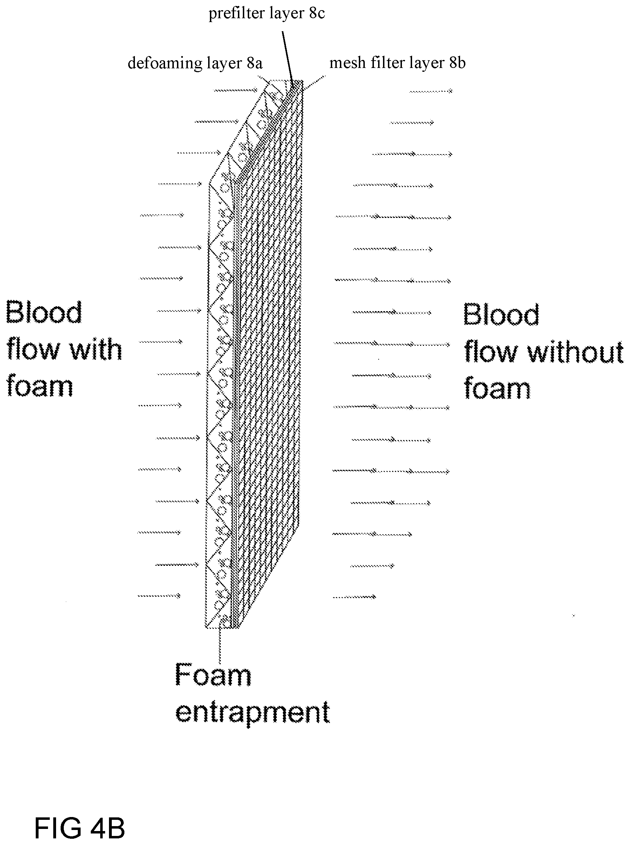

The defoaming layer 8a is made of an open mesh fabric with a three-dimensional (3D), embossed structure for foam entrapment. In the case of the embodiment shown in Figure 4A (and also in Figure 4B) the spatial structure of the defoaming layer is a diamond pattern with regularly arranged protrusions and depressions of a certain height. This structure or weave pattern is also known as Gauffree Diamond. The depressions form cells for capturing the formed foam of the biological fluid.

Defoaming layer 8a may have a thickness greater than the thickness of the downstream pre-filter layer 8c or mesh layer 8b. In an embodiment, defoaming layer 8a may have a thickness greater than the thickness of 2 to 3 filaments. The three-dimensional structure and thickness of the defoaming layer means that the pores in the fabric are not all within the same plane.

The fiber material of the defoaming layer is polypropylene (PP) with fiber diameters between 200-300 µm, such as 200, 250, 300 µm. The mesh count is between 12-16 n/cm. The mesh size or opening is between 250-300 µm.

The mesh filter layer 8b is made of interconnecting threads forming a grid or a net. The mesh size of mesh filter material 8b is between 40 µm and 120 µm and thus smaller than the mesh size of the defoaming layer. In case of the embodiment shown in Figure 4A wherein the filters system consists only of the defoaming layer and the mesh filter layer the mesh size of the mesh filter material may be 120 µm. Such a filter system is applicable for large pore size filtration.

The filter system as shown in Figure 8B consists of three layers: defoaming layer 8a, mesh filter layer 8b and a prefilter layer 8c arranged (or sandwiched) between defoaming layer 8a and mesh filet layer 8b.

The prefilter layer 8c consists of non-woven fibers with a trilobal cross-section, whereas the mesh filter layer 8b consists of a regularly formed medical grade mesh.

In case of the embodiment shown in Figure 4B wherein the filters system consists three layers (defoaming layer 8a, prefilter layer 8c and mesh filter layer 8b) the mesh size of the mesh filter material may be 40 µm. Such a filter system is applicable for low pore size filtration.

As depicted in both Figures 4A and 4B the blood flow enters the filter system 8 through the defoaming layer 8a. The foam bubbles contained in the blood are entrapped within the 3D structure of the defoaming layer 8a and are thus prevented to enter the downstream prefilter layer 8c and the mesh filter layer 8b. The blood flow leaving the filter system on the mesh filter side is foam free.

The pressure depends on the pore size of the material used and from its basis weight. The pressure is determined by Capillary flow porometry. This method allows for determining the pore size of the filter material (MFP, Mean Flow Pore).

## Claims

1. Filter assembly for filtering a blood comprising a filter system (8) and a filter holder (7),
- wherein the filter system (8) and the filter holder (7) are in contact with each other,
- wherein the filter system consists of at least two layers,
- wherein the first layer is a defoaming layer (8a) made of a monofilament woven open mesh fabric and with an embossed three-dimensional structure configured for entrapping foam built up in the blood,
• wherein said defoaming layer (8a) includes pores distributed in a nonplanar arrangement;
- wherein the second layer is a mesh filter layer (8b), and
- wherein the mesh filter layer (8b) is arranged downstream of the defoaming layer (8a).

2. Filter assembly according to claim 1, **characterized in that** the defoaming layer (8a) comprises a mesh opening between 100 - 500 µm, in particular between 150 - 400 µm, in particular between 200 - 350 µm, and more particularly between 250-300 µm.

3. Filter assembly according to one of the preceding claims, **characterized in that** the defoaming layer (8a) is made of monofilament fibers with a diameter between 100-350 µm, in particular 150 - 300 µm, more particularly 200-300 µm.

4. Filter assembly according to one of the preceding claims, **characterized in that** the defoaming layer (8a) has a mesh count between 10-50 n/cm, in particular between 10- 30 n/cm, more particularly between 10-20 n/cm.

5. Filter assembly according to one of the preceding claims, **characterized in that** the mesh filter layer (8b) has a mesh size in a range of between 20 and 150 µm, in particular between 30 and 140 µm, in particular between 40 and 130 µm, in particular between 50 and 120 µm, in particular between 60 and 110 µm, in particular between 70 and 100 µm, in particular between 80 and 90 µm.

6. Filter assembly according to one of the preceding claims, wherein the filter assembly further comprises a prefilter layer (8c) arranged downstream of the defoaming layer (8a) and upstream of the mesh filter layer (8b), wherein said mesh filter layer has a mesh size of approximately 40 µm, wherein the prefilter layer (8c) comprises preferably a spunbound nonwoven fabric

7. Filter assembly according to any one of claims 1 through 5 wherein the filter assembly does not include a pre-filter, wherein said mesh filter layer has a mesh size of approximately 120 µm.

8. Filter assembly according to claim 6 or 7, **characterized in that** the pore size of the prefilter layer (8c) is in a range of between 20 and 150 µm, in particular between 30 and 140 µm, in particular between 40 and 130 µm, in particular between 50 and 120 µm, in particular between 60 and 110 µm, in particular between 70 and 100 µm, in particular between 80 and 90 µm.

9. Filter assembly according to any of the preceding claims, **characterized in that** the filter holder is made from plastic and is over-molded over at least a part of the filter system, wherein said plastic is preferably selected from ABS or polycarbonate.

10. Filter assembly according to any of the preceding claims, **characterized in that** the filter assembly is free of anti-foam agents

11. Filter assembly of any one of the preceding claims wherein said defoaming layer has a thickness of 150-650 pm, in particular 200-500 µm, in particular 250-400 µm, in particular 300-350 µm.

12. Use of a filter assembly according to any of the preceding claims for filtering blood ex vivo.

13. Container for collecting blood comprising a filter assembly according to any of claims 1 to 12 as a filter module.

14. Container according to claim 13, further comprising a container housing (2) with a body fluid inlet (4) though which body fluid can enter an inlet section (5) of the container housing (2), a body fluid collection section (9), a vacuum connector(10) for connecting a vacuum source to the container housing (2) for applying a low pressure to the inlet section (5) and the body fluid collection section (9), and the filter assembly as filter module (6) being arranged between the inlet section (5) and the body fluid collection section (9), such that the defoaming layer faces the inlet section (5) and the mesh filter layer faces the body fluid collection section (9).

15. Container according to claim 13, **characterized in that** the container housing (2) additionally comprises a hydrophobic filter (11) that is arranged between the body fluid collection section (9) and the vacuum connector (10) in flow direction of air drawn by a vacuum source from the body fluid collection section (9) during intended use of the container.

## Patentansprüche

1. Filteranordnung zum Filtern von Blut, die ein Filtersystem (8) und einen Filterhalter (7) umfasst,
- wobei das Filtersystem (8) und der Filterhalter (7) miteinander in Kontakt stehen,
- wobei das Filtersystem aus zumindest zwei Schichten besteht,
- wobei die erste Schicht eine Entschäumungsschicht (8a) aus einem monofilen gewebten offenmaschigen Gewebe und mit einer dreidimensionalen erhabenen Struktur ist, die derart konfiguriert ist, dass sie in dem Blut angesammelten Schaum auffängt,
• wobei die Entschäumungsschicht (8a) Poren aufweist, die in einer nicht-planaren Anordnung verteilt sind;
- wobei die zweite Schicht eine Maschenfilterschicht (8b) ist, und
- wobei die Maschenfilterschicht (8b) stromabwärts von der Entschäumungsschicht (8a) angeordnet ist.

2. Filteranordnung nach Anspruch 1, **dadurch gekennzeichnet, dass** die Entschäumungsschicht (8a) eine Maschenweite zwischen 100 - 500 µm, insbesondere zwischen 150 - 400 µm, insbesondere zwischen 200 - 350 µm, und besonders bevorzugt zwischen 250 - 300 µm aufweist.

3. Filteranordnung nach einem der vorstehenden Ansprüche, **dadurch gekennzeichnet, dass** die Entschäumungsschicht (8a) aus monofilen Fasern mit einem Durchmesser zwischen 100 - 350 µm, insbesondere 150 - 300 µm, besonders bevorzugt 200 - 300 µm, gebildet ist.

4. Filteranordnung nach einem der vorstehenden Ansprüche, **dadurch gekennzeichnet, dass** die Entschäumungsschicht (8a) eine Maschenanzahl zwischen 10-50 n/cm, insbesondere zwischen 10-30 n/cm, besonders bevorzugt zwischen 10-20 n/cm aufweist.

5. Filteranordnung nach einem der vorstehenden Ansprüche, **dadurch gekennzeichnet, dass** die Maschenfilterschicht (8b) eine Maschengröße in einem Bereich zwischen 20 und 150 µm, insbesondere zwischen 30 und 140 µm, insbesondere zwischen 40 und 130 µm, insbesondere zwischen 50 und 120 µm, insbesondere zwischen 60 und 110 µm, insbesondere zwischen 70 und 100 µm, insbesondere zwischen 80 und 90 µm aufweist.

6. Filteranordnung nach einem der vorstehenden Ansprüche, wobei die Filteranordnung ferner eine Vorfilterschicht (8c) aufweist, die stromabwärts der Entschäumungsschicht (8a) und stromaufwärts der Maschenfilterschicht (8b) angeordnet ist, wobei die Maschenfilterschicht eine Maschengröße von etwa 40 µm aufweist, wobei die Vorfilterschicht (8c) bevorzugt einen Spinnvliesstoff umfasst.

7. Filteranordnung nach einem der Ansprüche 1 bis 5, wobei die Filteranordnung keinen Vorfilter aufweist, wobei die Maschenfilterschicht eine Maschengröße von etwa 120 µm aufweist.

8. Filteranordnung nach Anspruch 6 oder 7, **dadurch gekennzeichnet, dass** die Porengröße der Vorfilterschicht (8c) in einem Bereich zwischen 20 und 150 µm, insbesondere zwischen 30 und 140 µm, vor allem zwischen 40 und 130 µm, insbesondere zwischen 50 und 120 µm, vor allem zwischen 60 und 110 µm, insbesondere zwischen 70 und 100 µm, vor allem zwischen 80 und 90 µm liegt.

9. Filteranordnung nach einem der vorstehenden Ansprüche, **dadurch gekennzeichnet, dass** der Filterhalter aus Kunststoff gebildet ist und auf zumindest einen Teil des Filtersystems aufgespritzt ist, wobei der Kunststoff bevorzugt aus ABS oder Polycarbonat ausgewählt ist.

10. Filteranordnung nach einem der vorstehenden Ansprüche, **dadurch gekennzeichnet, dass** die Filteranordnung frei von Antischaummitteln ist.

11. Filteranordnung nach einem der vorstehenden Ansprüche, wobei die Entschäumungsschicht eine Dicke von 150 - 650 pm, insbesondere von 200 - 500 µm, insbesondere von 250 - 400 µm, insbesondere von 300 - 350 µm aufweist.

12. Verwendung einer Filteranordnung nach einem der vorstehenden Ansprüche zum Filtern von Blut ex vivo.

13. Behälter zum Sammeln von Blut, umfassend eine Filteranordnung nach einem der Ansprüche 1 bis 12 als Filtermodul.

14. Behälter nach Anspruch 13, ferner aufweisend ein Behältergehäuse (2) mit einem Körperfluideinlass (4), durch den Körperfluid in einen Einlassabschnitt (5) des Behältergehäuses (2) eintreten kann, einen Körperfluidsammelabschnitt (9), einen Vakuumanschluss (10) zum Verbinden einer Vakuumquelle mit dem Behältergehäuse (2), um einen niedrigen Druck auf den Einlassabschnitt (5) und den Körperfluidsammelabschnitt (9) aufzubringen, und die Filteranordnung als Filtermodul (6), die zwischen dem Einlassabschnitt (5) und dem Körperfluidsammelabschnitt (9) angeordnet ist, so dass die Entschäumungsschicht dem Einlassabschnitt (5) zugewandt ist und die Maschenfilterschicht dem Körperfluidsammelabschnitt (9) zugewandt ist.

15. Behälter nach Anspruch 13, **dadurch gekennzeichnet, dass** das Behältergehäuse (2) zusätzlich einen hydrophoben Filter (11) aufweist, der zwischen dem Körperfluidsammelabschnitt (9) und dem Vakuumanschluss (10) in einer Strömungsrichtung von Luft angeordnet ist, die von einer Vakuumquelle des Körperfluidsammelabschnitts (9) während der beabsichtigten Verwendung des Behälters angesaugt wird.

## Revendications

1. Ensemble filtre destiné à filtrer un sang comprenant un système de filtre (8) et un support de filtre (7),
- dans lequel le système de filtre (8) et le support de filtre (7) sont en contact l'un avec l'autre,
- dans lequel le système de filtre est constitué d'au moins deux couches,
- dans lequel la première couche est une couche de démoussage (8a) constituée d'un tissu à mailles ouvertes tissées monofilamentaires et avec une structure tridimensionnelle gaufrée configurée pour piéger de la mousse accumulée dans le sang,
• dans lequel ladite couche de démoussage (8a) comporte des pores répartis selon une disposition non planaire ;
- dans lequel la deuxième couche est une couche de filtre à mailles (8b), et
- dans lequel la couche de filtre à mailles (8b) est disposée en aval de la couche de démoussage (8a).

2. Ensemble filtre selon la revendication 1, **caractérisé en ce que** la couche de démoussage (8a) comprend une ouverture de maille entre 100-500 µm, en particulier entre 150-400 µm, en particulier entre 200-350 µm, et plus particulièrement entre 250-300 µm.

3. Ensemble filtre selon l'une quelconque des revendications précédentes, **caractérisé en ce que** la couche de démoussage (8a) est constituée de fibres monofilamentaires avec un diamètre entre 100-350 µm, en particulier 150300 µm, plus particulièrement 200-300 µm.

4. Ensemble filtre selon l'une quelconque des revendications précédentes, **caractérisé en ce que** la couche de démoussage (8a) présente un décompte de mailles entre 10-50 n/cm, en particulier entre 10-30 n/cm, plus particulièrement entre 10-20 n/cm.

5. Ensemble filtre selon l'une quelconque des revendications précédentes, **caractérisé en ce que** la couche de filtre à mailles (8b) présente une taille de maille dans une plage entre 20 et 150 µm, en particulier entre 30 et 140 µm, en particulier entre 40 et 130 µm, en particulier entre 50 et 120 µm, en particulier entre 60 et 110 µm, en particulier entre 70 et 100 µm, en particulier entre 80 et 90 µm.

6. Ensemble filtre selon l'une quelconque des revendications précédentes, dans lequel l'ensemble filtre comprend en outre une couche de préfiltre (8c) disposée en aval de la couche de démoussage (8a) et en amont de la couche de filtre à mailles (8b), dans lequel ladite couche de filtre à mailles présente une taille de maille d'approximativement 40 µm, dans lequel la couche de préfiltre (8c) comprend de manière préférée un tissu non-tissé filé-lié.

7. Ensemble filtre selon l'une quelconque des revendications 1 à 5, dans lequel l'ensemble filtre ne comporte pas un préfiltre, dans lequel ladite couche de filtre à mailles présente une taille de maille d'approximativement 120 µm.

8. Ensemble filtre selon la revendication 6 ou 7, **caractérisé en ce que** la taille de pore de la couche de préfiltre (8c) se situe dans une plage entre 20 et 150 µm, en particulier entre 30 et 140 µm, en particulier entre 40 et 130 µm, en particulier entre 50 et 120 µm, en particulier entre 60 et 110 µm, en particulier entre 70 et 100 µm, en particulier entre 80 et 90 µm.

9. Ensemble filtre selon l'une quelconque des revendications précédentes, **caractérisé en ce que** le support de filtre est constitué de plastique et est surmoulé sur au moins une partie du système de filtre, dans lequel ledit plastique est choisi de manière préférée parmi l'ABS ou le polycarbonate.

10. Ensemble filtre selon l'une quelconque des revendications précédentes, **caractérisé en ce que** l'ensemble filtre est sans agents antimousse.

11. Ensemble filtre selon l'une quelconque des revendications précédentes, dans lequel ladite couche de démoussage présente une épaisseur de 150-650 pm, en particulier 200-500 µm, en particulier 250-400 µm, en particulier 300-350 µm.

12. Utilisation d'un ensemble filtre selon l'une quelconque des revendications précédentes pour filtrer du sang ex vivo.

13. Récipient pour collecter du sang, comprenant un ensemble filtre selon l'une quelconque des revendications 1 à 12 en tant qu'un module de filtre.

14. Récipient selon la revendication 13, comprenant en outre un boîtier de récipient (2) avec une entrée de fluide corporel (4), par laquelle du fluide corporel peut entrer dans une section d'entrée (5) du boîtier de récipient (2), une section de collecte de fluide corporel (9), un raccord sous vide (10) pour raccorder une source sous vide au boîtier de récipient (2) pour appliquer une pression basse sur la section d'entrée (5) et la section de collecte de fluide corporel (9), et l'ensemble filtre en tant que module de filtre (6) étant disposé entre la section d'entrée (5) et la section de collecte de fluide corporel (9) de telle sorte que la couche de démoussage est tournée vers la section d'entrée (5) et la couche de filtre à mailles est tournée vers la section de collecte de fluide corporel (9).

15. Récipient selon la revendication 13, **caractérisé en ce que** le boîtier de récipient (2) comprend en supplément un filtre hydrophobe (11) qui est disposé entre la section de collecte de fluide corporel (9) et le raccord sous vide (10) dans une direction d'écoulement d'air aspiré par une source sous vide depuis la section de collecte de fluide corporel (9) pendant l'utilisation prévue du récipient.
